# EUROPEAN PATENT SPECIFICATION

(11) **EP 1 246 650 B1**
(45) Date of publication and mention of the grant of the patent: **17.09.2003**
(21) Application number: 00985550.3
(22) Date of filing: 13.12.2000
(51) Int. Cl.: A61L 15/28

(54) **FLEXIBLE CHITOSAN FILM FOR WOUND DRESSINGS**
FLEXIBLER CHITOSANFILM ZUR VERWENDUNG ALS WUNDABDECKUNG
FILM DE CHITOSANE SOUPLE POUR PANSEMENTS

(30) Priority: 13.12.1999 GB 9929472
(43) Date of publication of application: 09.10.2002
(73) Proprietor: Kordestani, Soheila Salahshoor, Tehran, Iran(Islamic Republic of) (IR)
(72) Inventor: Kordestani, Soheila Salahshoor, Tehran, Iran(Islamic Republic of) (IR)
(74) Representative: Matthews, Derek Peter
(86) International application number: GB0004777
(87) International publication number: WO01041820

(56) References cited:
- FR-A- 2 701 266
- US-A- 5 597 581
- US-A- 5 836 970
- US-A- 5 902 798

## Description

### Background of the invention

### 1. Field of the invention

This invention relates to a polymeric film suitable for use as a wound dressing.

### 2. Description of the related art

There has been a long-felt want for a polymeric film for dressing wounds, particularly for patients suffering from burns. A dressing which imparts to the wound a high degree of resistance to infection, adheres closely to the wound, can absorb water from it, yet does not have to be changed, would be highly desirable.

### Summary of the invention

The invention includes a flexible polymeric film, suitable for use as a wound dressing and biodegradable when placed in contact with the wound, wherein the polymer comprises at least 80% by weight of chitosan, the chitosan has a degree of deacetylation, reckoned by reference to chitin, of 70-88%, the average molecular weight of the chitosan (determined from viscosity or gel permeation chromatography) is about 300,000-1,000,000, and the film has a water absorbency of from 500 to 1500 weight percent of the dry film.

Other aspects of the invention include a wound dressing comprising the film and the use of the film or a wound dressing comprising it for dressing a wound in a human or animal patient. It may be used by applying the film or wound dressing of the invention to the site of the wound, which may be an external or internal wound

The term "water absorbency" means water uptake determined by placing the dry film in distilled water, placing it on filter paper to remove excess water and determining the difference in weight between the dry and wet film. The "dry" starting film is allowed to equilibrate with the ambient atmosphere, before the test takes place.

The term "film", as used herein, is not limited to a self-supporting film and in particular includes a laminate having a layer of a polymeric film as defined above.

The term "polymeric film", as used herein, signifies that the film is based on a polymer, but it need not consist of polymer. It may contain, for example, a plasticiser (which is not necessarily a polymer), slip agent, filler, pigment, antibacterial agent, antiseptic agent for accelerating or retarding wound healing, or any other additive compatible with its intended use in contact with a wound.

The term "comprises" as used herein, means "consists of or includes". Thus, the polymer need not consist wholly of chitosan, but can include a minor proportion, e.g. 0.0001 to 20% by weight, of another polymer, for example a hydrophilic polymer imparting improved tensile strength to the film or even better adhesion of the film to the wound. Proportions are by total weight of polymer (chitosan plus added polymer).

The term "chitosan" means partially deacetylated chitin as defined above (100% deacetylation being impossible in practice). Thus, any units of (non-deacetylated) chitobiose (monomeric chitin) within the polymeric chains are considered to be part of the "chitosan" for the purposes of definition. In this invention the polymer preferably consists entirely of chitosan, but, in view of the above definition, it may contain chitin as part of the "chitosan".

### Brief Description of the Drawings

Figures 1-3 show, respectively, the X-ray diffraction spectra of samples of chitin and chitosan made in the process of the invention, both from Example 3, and of commercial chitosan Sample B; and
Figures 4 and 5 show the reduction in the rate of healing achieved when a chitosan film of the invention is used, compared with a control.

### Description of the preferred embodiments

The thickness of the film may be any appropriate to the intended purpose and is desirably in the range 5-50 micrometres, more preferably 10-30 micrometres.

It is believed that the key features of the film are its water absorbency, flexibility and tenacity.

The water absorbency of the film is measured by the maximum percentage water uptake by the dry film on exposure to the atmosphere. It is preferably within the range 500-1500% especially 500-1000% and most preferably 600-900%.

The preferred flexibility of the film may be defined as approximately within the range typically found in clear films used to wrap or cover foodstuffs in the domestic kitchen.

The tenacity of the film is preferably within the range 80-150 MPa, most preferably 100-130 MPa.

The average molecular weight of the chitosan, determined on the above basis, is preferably 300,000-700,000, most preferably 400,000-600,000.

Chitosan is frequently cross-linked to improve its strength, using e.g. epichlorhydrin. This is an optional feature in the present invention, to improve tensile strength.

Preferably chitosan is the sole polymeric component of the film, because it is biodegradable and imparts excellent water absorbency to the film. However, one or more other compatible polymers can be blended with the chitosan in forming the film, in a proportion of up to 50%, preferably up to 30%, especially up to 20% and most usually up to 10% by weight of the total polymer. Examples are gelatin, collagen, polyvinyl alcohol, polyvinyl pyrrolidone, polyethylene glycol or a hydrophilic polymer of acrylic or methacrylic acid.

The use of chitosan as a wound healing agent is known, but hitherto the art has not found how to make a good film from it. The film of the invention gives a better rate of wound healing in a patient than chitosan powder, and a reduction in scarring. It is non-toxic, bactericidal against many important bacteria, and highly water-absorbent.

The film normally adheres well to a wound, but is best kept in place by other means, e.g. a covering such as a small bandage. To remove the dressing from an external wound, after healing, it is best to wait until the wound is dry. It can then be removed as integral film. An alternative would be to wash it off in saline solution. However, the film will usually be absorbed into and biodegraded in the wound. Thus, it may be used in treating internal wounds (including lesions and cuts). The film is most suitable for treating relatively shallow wounds, especially wounds having the depth typical of a burn wound. It is particularly applicable to burn wounds, where good protection from infection is needed.

In the preparation of the film from chitosan, it is convenient to cast it and for this purpose the casting plate or roller is preferably lubricated with a polysiloxane. When using such a hydrophobic lubricant, the film will acquire a thin surface layer of it. It is then best applied to the wound with this hydrophobic face upwards and the non-lubricated face in contact with the wound. The hydrophobic lubricant can reduce the water absorbency by some 10-15%.

In the process of preparing the film of the invention, the source of chitin may be any which produces α-chitin, and is preferably the shells of crustaceans, most preferably shrimps. Other crustaceans such as lobsters and crabs can be used. Preferably the shrimps are predominantly banana shrimps, *Penaeus merguiensis*, obtainable from the Sea of Oman in the Persian Gulf. The objectives of the process, generally stated, are to avoid harsh conditions, especially high temperatures or, except in the deacetylation step, extremes of pH. Thus, the shells are dried at a moderate temperature (30-50°C), e.g. simply in air or in an oven. Whereas, conventionally, alkali is used to remove protein from the ground shells, the acidification step comes first in the process of the invention. This is believed to produce chitin of higher purity. Any acid can be used which demineralises the shells, removing calcium salts such as carbonates and phosphates. Preferably the acid used is 0.4 to 3N HCl, especially about 0.5N to 1.5N HCl, in contrast to 2N used previously. The acid treatment is preferably carried out for up to 2 to 5 hours, especially 2 to 3 hours. Protein is then removed with mild alkali at a relatively low temperature, e.g. 0.4 to 3N NaOH or KOH, especially 0.5N to 1.5N, and at a temperature preferably below 70°C especially 50-70°C and for a time preferably not longer than 24 hours, especially 3 to 24 hours.

Chitin and chitosan prepared in this invention typically have an alpha-chitin crystalline structure. The preferred products typically have a significant X-ray diffraction peak at a value of 2θ greater than 21°, e.g. between 22 and 27°, most preferably 26-27°, a feature which is believed new, i.e. not observed in chitosan of the prior art. The significant X-ray diffraction peak has an intensity ratio I/Iₒ (where Iₒ is the intensity of the peak of greatest intensity) of at least 5%, typically at least 15%. Although normally of relatively low intensity, it is consistently observed.

Deacetylation of the chitin, preferably to a value in the range 80-87% deacetylation, can be carried out by using 8M to 15M, preferably about 12 to 13M, NaOH. It has been found desirable to exclude air, to obtain chitosan of high molecular weight and crystallinity and thus impart higher tensile strength to the film. Preferably the deacetylation is conducted in an atmosphere of non-oxidising gas such as nitrogen. The deacetylation preferably carried out at a temperature of from 25 to 105°C for a period of up to an hour, e.g. 15 minutes to 1 hour, depending on temperature.

The following Examples illustrate the invention.

### Example 1

### A. Production of chitin

Shrimps were freshly collected from the Persian Gulf. The shells of the bodies and heads of shrimps were separated, washed under running water to remove sand and other particles and sun-dried or dried at 40°C. (In many production methods of the prior art, the shells are dried at higher temperatures, about 100°C. This affects the structure of the resulting chitin). The shells were ground to a powder of about 300 micrometres. 132 grams of the ground shells were digested in 2 litres of 1 N hydrochloric acid at 25°C for 3 hours. The total amount of the acid was added in two steps with occasional stirring. These are relatively mild conditions. A higher concentration of hydrochloric acid drastically affects the structure of the chitin and consequent chitosan produced.

The acid was decanted and the material was washed with distilled water. In this demineralisation stage, removal of calcium carbonate and calcium phosphate was completed.

To remove proteins, the material prepared above was soaked in 3 litres of 1 N sodium hydroxide at 65°C for 24 hours, with occasional stirring. The liquid phase was decanted and the resulting chitin was washed several times with distilled water. The yield was between 10% to 15% of the dried shells. The Fourier Transform InfraRed (FTIR) spectrum showed absorption bands at about 1656, 1552 and 1313 cm⁻¹, with bands at 1650 and 1620 cm⁻¹ characteristic of α-chitin.

The chitin produced had the following properties:

| | |
|---|---|
| Moisture content | 8.5% |
| Ash at 900°C | 0.94% |
| Calcium content | 0.0981% |
| X-ray diffraction peaks | 2θ = about 19° (intense, common to many forms of chitin) and also an additional peak at a higher value of 2θ, above 21° (relatively weak, not seen in ordinary forms of chitin). |
| Viscosity of 1% (w/v) solution in N,N-dimethylacetamide/LiCI at 25°C | 500-1500 cps |

### B. Production of chitosan

33 grams of chitin were treated with 1 litre of 50% (500g/litre) NaOH and 10ml of ethanol (ethanol was used for decoloration) at 100°C for 1 hour under nitrogen. The liquid phase was discarded and the chitosan was collected and washed with distilled water several times. Using 50% caustic soda has produced chitosan with a degree of deacetylation of about 85%, by reference to chitin.

The mild procedures used resulted in a form of chitin that possibly retains its native ultrastructure and certainly imparts a very large surface area. In the resulting chitosan, chain fragmentation in the chitin produced is very limited.

| | |
|---|---|
| Degree of deacetylation, estimated by elemental analysis and NMR | about 85% |
| Relative molecular weight (of the chitosan) | 400,000 |
| Viscosity of 1% (w/v) solution in acetic acid at 25°C | 500 - 1500cps |

### C. Preparation of chitosan film

Chitosan was dissolved in 1.5% acetic acid and stirred until it dissolved completely. The solution was filtered and spread on a casting plate, coated with medical grade polysiloxane at a thickness of 0.02 mm (20 micrometres). The spread film was allowed to dry in air to a dried thickness of 12 micrometres. The polysiloxane gave better mechanical properties to the film, eliminating the need for an epichlorohydrin cross-linking agent. Also it made it slightly more viscoelastic.

The water absorbency of the film was 700-800% (weight/weight based on the dry film), measured as described above.

### D. Animal tests

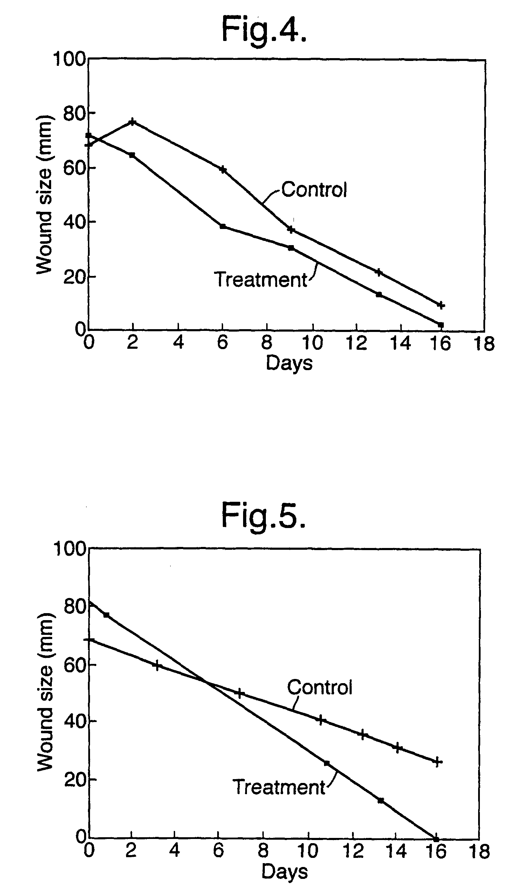

Two squares full-thickness skin defects measuring 1 cm² were made bilaterally around a sterile glass template on the back of 54 guinea pigs of either sex with approximately 400g body weight. The animals were randomly divided into six groups of nine. The left wounds of each group were treated with chitins and chitosans in powder form, and the film made from chitosan by the above procedure respectively. The right wound of each group of animal served as control. The non-pressure and non-adhesive bandage of each wound was changed every other day for clinical assessment and photography. Histopathological evaluations were carried out on samples taken from three guinea pigs of each group at the end of the first, second and third week of the trial. Quantitative assessment of changes in wound size revealed that rate of healing in wounds treated with the film made from chitosan was significantly higher than the control wounds. See Figure 4 in which wound size in mm is plotted on the y-axis and healing time in days on the x-axis. The line marked "+" is for the control and that marked "•" is for the chitosan film of the invention. In the wounds treated with the chitosan film of the invention, cosmetic appearance was superior, and differences in contraction and re-epithelialisation rates were significant; also fibroblasts and newly formed capillaries proliferated more prominently than in the control. The statistical method used was the T test and the software package was Sistat. In Figure 5, best fit lines were produced from the equations of the regression lines y = 73.16 - 4.694x (treatment) and y = 80.46 - 4.34x (control).

### Examples 2 - 4

### Comparative Study of Different Chitosan Films

The aim of this study was to compare the characteristics of two different chitosan films, suitable for wound dressing, prepared from various sources. To this end, chitosans with low, medium and high molecular weights were prepared from 100 percent fresh shrimp shells from the Persian Gulf. Films were prepared by a process of the invention from three grades of these chitosans and three commercially available chitosans, herein designated A, B and C, with high, medium and low molecular weights respectively. Results showed that chitosans in powder form prepared by the process of the invention gave compared to different X-ray diffraction patterns from the commercially available chitosans. Also the chitosan films of the invention exhibited superior water uptake and tensile strength than film from the commercially available chitosans. These resultsconfirm the suitability of the film of the invention for use in wound dressing.

### A. Preparation of chitosan

The shrimps collected consisted of a mixture of 90% *Penaeus merguiensis* (banana shrimp) and 10% other species in which *Meta Penaeus caffinis* (white shrimp) and *Panaeus semi sulcatus* (Green tiger shrimp) predominated.

The head and body shells of the shrimps were soaked in distilled water for 30 mins to remove all the sands and other impurities. The liquid was decanted and shells were dried in overn at 40°C. The shells were micromilled to a powder of 250 micrometres (60 mesh).

The ground shells (226g) were soaked in 0.5N HCI (Ex. 2), IN HCI (Ex. 3) or 3N HCl (Ex. 4) for 3 hours at room temperature. The total volume of acid was added in three stages. The suspension was stirred occasionally. The acid was decanted and the remaining solid was filtered and washed, several times, with distilled water and dried at 40°C. In Example 2 (but not in Examples 3 and 4), the decantation, filtration and washing were repeated, to increase the purity of the material. The shells were treated with 0.5N NaOH (Ex. 2), IN NaOH (Ex. 3) or 3N NaOH (Ex. 4) for 24 hours at 65°C in a covered container. The liquid was decanted and the solid particles were washed, several times, with acetone and distilled water, filtered completely and dried in an oven at 40°C.

### B. Preparation of chitosan

The chitin was treated with 50% NaOH using 1% (v/v) ethanol under nitrogen at boiling temperature for 1-2 hours. The liquid was decanted and the solid was washed, several times, with acetone and distilled water.

### C. Preparation of chitosan film

Chitosan (1g) was dissolved in 1% acetic acid. The dissolved solutions were stirred for 4-5 hours before filtration and degassing. The films were cast on a laboratory scale film-casting plate (30 x 50 cm) and left to dry in a fume cupboard for 24 hours at room temperature.

For comparison, film was prepared by the same method from three samples of commercial chitosan.

### D. Analytical tests

Analytical tests were designed in such a manner that firstly to be able to determine the most important characteristics of the chitins and chitosans produced from the Iranian shrimps and secondly to compare the suitability of the films, prepared from the two different chitosans, for wound dressing. To achieve the second aim tests such as ash, heavy metal content, moisture, water uptake and tensile properties of the films were carried out.

### Results

### Fourier transform infra-red spectroscopy

FTIR spectroscopy was carried out on the chitin and chitosan of Example 3 only. The chitin showed two bands at about 1655 and 620 cm⁻¹, indicating the hydrogen bonded amide which is characteristic of chitin. The chitosan spectrum showed two bands at about 1650 and 1580 cm⁻¹ which indicate the substitution of acetamide group by the amino group.

### Ash content

The residue on ignition to ash at 900°C of chitin from Examples 2-4 and chitosan from Example 3 was less than 0.9%.

### Moisture Regain

The chitin and chitosan samples of Examples 2-4 were tested for moisture regain. Samples (1g) were dried at 100°C for 2 hours and were then conditioned at 25°C and RH 65 ± 2% for 24 hours. The moisture regains, determined as the percentage weight increase of the dried film after conditioning, for the samples of Examples 2-4 were respectively as follows:

| | | | | |
|---|---|---|---|---|
| Chitin | 6.8, | 6.2, | 7.5 | % |
| Chitosan | 6.6, | 7.6, | 7.6 | % |

### Degree of Deacetylation

This was determined, for the chitosan of Example 3 only, using NMR, as 81.8% deacetylation. This is a normal figure. Commercial chitosan typically has a degree of deacetylation in the 80-86% range.

### Viscosity

The viscosities of the chitosan samples, determined by Brookfield viscometer using 1% solutions in 1% acetic acid at 25°C, were respectively as follows:

| | | | |
|---|---|---|---|
| Examples 2-4: | 250, | 100, | 10 |
| Commercial Chitosans A, B and C: | 754, | 115, | 12 |

These results indicate that two of the three commercial samples are well matched for molecular weight with the samples of Examples 3 and 4, while the other commercial sample is of higher molecular weight than that of Example 2

### X-ray diffraction

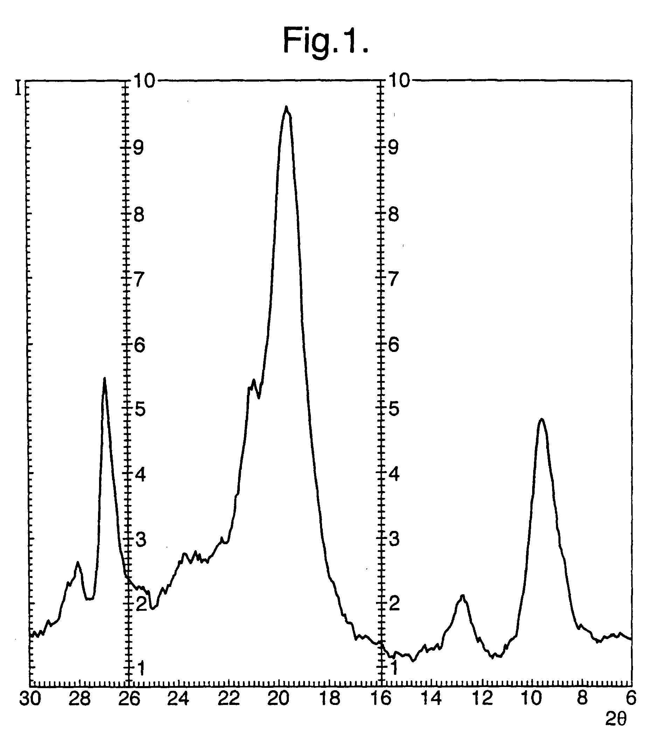

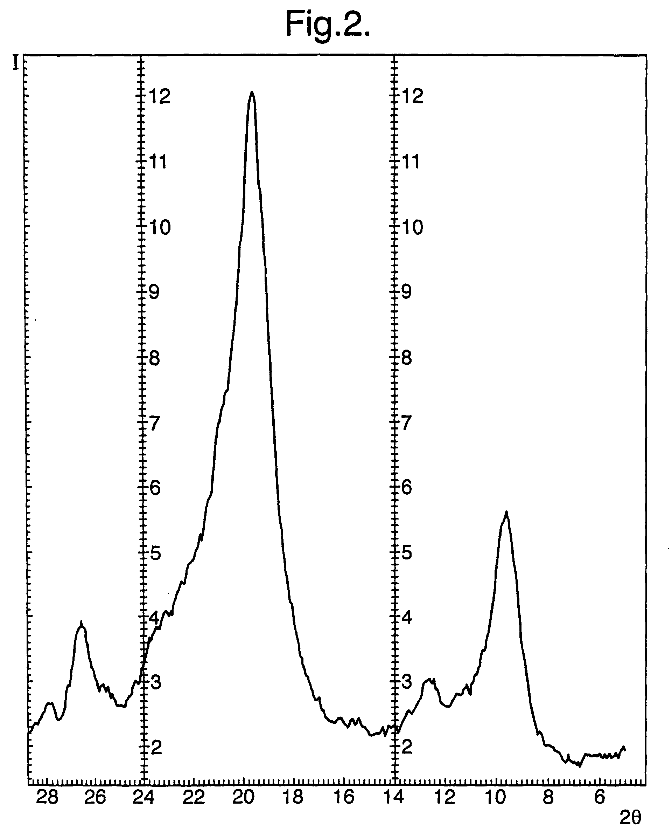

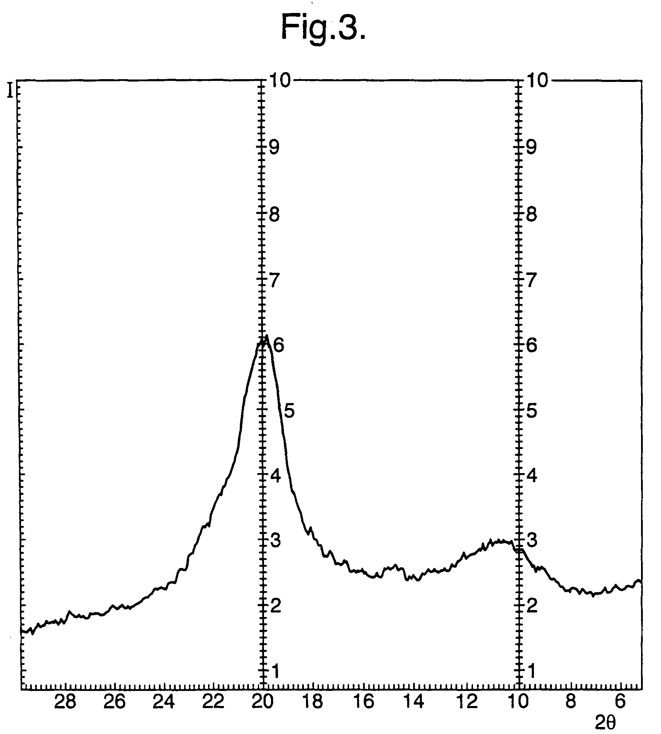

Figures 1-3 show, respectively, the X-ray diffraction spectra of samples of chitin and chitosan made in the process of the invention, both from Example 3, and of commercial chitosan Sample B.

Intensity is plotted on the y-axis against the angle of diffraction, 2θ, on the x-axis. The positions of the peaks are presented in Table 1. The intensity (I = peak height) is given as a percentage of the highest value (Io).

**Table 1:**

| X-ray diffraction spacings etc. for chitin and chitosan | | | |
|---|---|---|---|
| Sample | 2θ° | D (Angstroms)* | I/Io (%) |
| Chitin, Ex. 3 | 9.5 | 9.30 | 44 |
| | 12.8 | 6.92 | 12 |
| | 19.6 | 4.53 | 100 |
| | 20.9 | 4.25 | 52 |
| | 26.8 | 3.31 | 52 |
| Chitosan, Ex. 3 | 9.6 | 9.20 | 37 |
| | 12.6 | 7.02 | 13.8 |
| | 19.6 | 4.53 | 100 |
| | 20.8 | 4.29 | 54 |
| | 26.6 | 3.35 | 21.8 |
| Commercially available chitosan "B" | 9.1 | 9.70 | 15.4 |
| | 10.6 | 8.35 | 25 |
| | 14.7 | 6.02 | 15.4 |
| | 19.8 | 4.48 | 100 |

| | | | |
|---|---|---|---|
| *Calculated from 2θ values using Bragg's law | | | |

The commercial chitosan sample shows a clearly different X-ray diffraction pattern from those of the chitin and chitosan samples prepared in the invention, which exhibit an extra peak at 2θ = 26°6' -26°8'. A. A. R. Muzzarelli "The polysaccharides", vol. 3, 413-450, Academic Press, 1985 has reported the typical peaks of chitosan as at 20 = 8°58' - 10°26' and 19°58' -20°00.

### Heavy Metals

The heavy metal content of the chitosan sample of Example 3 was determined to be less than 0.001%. This figure is considered normal.

### Water Uptake (Absorbency)

The water uptakes of the films of Examples 2-4 and from commercial chitosans A, B and C were measured as described previously, with the results shown in Table 2 below. Each test was carried out three times. The figures given are averages.

**Table 2:**

| Water Uptake of chitosan films | | | |
|---|---|---|---|
| Samples | Wt. of Dry Film (g) | Wt. of Wet Film (g) | Water Uptake % |
| Ex. 2 | 0.050 | 0.350 | 600 |
| Ex. 3 | 0.060 | 0.590 | 883 |
| Ex. 4 | 0.035 | 0.250 | 614 |
| A | 0.045 | 0.095 | 106 |
| B | 0.038 | 0.088 | 142 |
| C | 0.038 | 0.120 | 194 |

The chitosan films of the invention show a greatly superior water uptake to those of the commercial samples of chitosan. In wound management the ability of a dressing to absorb water from the wound is considered important.

### Tensille Properties

The tensile properties of 0.02 mm thick films of Examples 2-4 and from commercial chitosans A, B and C, including thickness, load at break, elongation at break and tenacity were measured for each sample. See Table 3. Each test was carried out five times. The figures given are averages.

**Table 3:**

| Tensile properties of chitosan films | | | |
|---|---|---|---|
| Sample | Load at Break (Newtons) | Elongation at Break (%) | Tenacity (Mpa) |
| Ex. 2 | 10.3 | 10.4 | 128.7 |
| Ex. 3 | 7.9 | 10.6 | 98.8 |
| Ex. 4 | 6.7 | 11.7 | 83.5 |
| A | 6.6 | 8.5 | 82.7 |
| B | 5.5 | 9.8 | 69.4 |
| C | 4.6 | 12.3 | 57.3 |

The films of the invention exhibited significantly greater tenacity than, and at least as good load at break as their commercial counterparts.

## Claims

1. A flexible polymeric film, suitable for use as a wound dressing and biodegradable when placed in contact with the wound, wherein the polymer comprises at least 80% by weight of chitosan, the chitosan has a degree of deacetylation, reckoned by reference to chitin, of 70-88%, the average molecular weight of the chitosan (determined from viscosity or gel permeation chromatography) is 300,000-1,000,000, and the film has a water absorbency of from 500 to 1500 weight percent of the dry film.

2. A film according to Claim 1, wherein the polymer consists entirely of chitosan.

3. A film according to Claim 1, wherein the average molecular weight of the chitosan is 400,000-600,000.

4. A film according to Claim 1, wherein the water absorbency is from 600 to 900 weight percent.

5. A film according to Claim 1, wherein the degree of deacetylation is from 80-87%.

6. A film according to Claim 1, having a tenacity of 80-150 MPa.

7. A film according to Claim 1, wherein the chitosan has an X-ray diffraction spectrum having a significant peak at a value of 2θ greater than 21°.

8. A wound dressing comprising a film defined in any preceding Claim.

## Patentansprüche

1. Flexibler Polymerisatfilm, geeignet zur Verwendung als Wundverband, der biologisch abbaubar ist, wenn er mit der Wunde in Kontakt gebracht wird, wobei das Polymerisat wenigstens 80 Gew.-% Chitosan enthält, das Chitosan einen Deacetylierungsgrad, bezogen auf Chitin, von 70-88 % hat, das mittlere Molekulargewicht des Chitosans (bestimmt aufgrund der Viskosität oder mit Hilfe der Gelpermeationschromatografie) 300.000-1.000.000 beträgt und der Film ein Wasserabsorptionsvermögen von 500 bis 1500 Gew.-% des trockenen Films hat.

2. Film nach Anspruch 1, worin das Polymerisat ganz aus Chitosan besteht.

3. Film nach Anspruch 1, worin das mittlere Molekulargewicht des Chitosans 400.000-600.000 beträgt.

4. Film nach Anspruch 1, worin das Wasserabsorptionsvermögen 600 bis 900 Gew.-% beträgt.

5. Film nach Anspruch 1,worin der Deacetylierungsgrad 80-87 % beträgt.

6. Film nach Anspruch 1 mit einer Reißfestigkeit von 80-150 MPa.

7. Film nach Anspruch 1, worin das Chitosan ein Röntgenbeugungsspektrum mit einem signifikanten Pik bei einem Wert von 2 θ größer als 21° hat.

8. Wundverband mit einem Film gemäß einem der vorhergehenden Ansprüche.

## Revendications

1. Film polymère flexible, utilisable en tant que pansement et biodégradable quand il est placé en contact avec une plaie, dans lequel le polymère comprend au moins 80 % en poids de chitosane, le chitosane a un degré de désacétylation, évalué par référence, à la chitine, de 70-88 %, la masse moléculaire moyenne du chitosane (déterminée à partir de la viscosité ou d'une chromatographie par perméation sur gel) est de 300 000 à 1 000 000, et le film a une capacité d'absorption d'eau de 500 à 1500 % en poids du film sec.

2. Film selon la revendication 1, dans lequel le polymère est entièrement constitué de chitosane.

3. Film selon la revendication 1, dans lequel la masse moléculaire moyenne du chitosane est de 400 000 à 600 000.

4. Film selon la revendication 1, dans lequel la capacité d'absorption d'eau est de 600 à 900 % en poids.

5. Film selon la revendication 1, dans lequel le degré de désacétylation est de 80 à 87 %.

6. Film selon la revendication 1, ayant une ténacité de 80 à 150 MPa.

7. Film selon la revendication 1, dans lequel le chitosane a un spectre de diffraction des rayons X ayant un pic significatif à une valeur de 2θ supérieure à 21°.

8. Pansement comprenant un film défini dans l'une quelconque des revendications précédentes.
